# EUROPEAN PATENT SPECIFICATION

(11) **EP 3 279 312 B1**
(45) Date of publication and mention of the grant of the patent: **31.07.2019**
(21) Application number: 17159099.5
(22) Date of filing: 03.03.2017
(51) Int. Cl.: C12N 1/20, A23C 9/123, A23C 9/12, A23C 13/16, A23L 3/3571, A23L 29/00, A01N 63/00, A01N 63/02, C12R 1/225

(54) **BIOPROTECTION OF DAIRY PRODUCTS**
BIOSCHUTZ VON MILCHPRODUKTEN
BIOPROTECTION DE PRODUITS LAITIERS

(30) Priority: 02.08.2016 EP 16182341
(43) Date of publication of application: 07.02.2018
(73) Proprietor: DSM IP Assets B.V., 6411 TE Heerlen (NL)
(72) Inventor: AUBERT, Cécile, 6100 AA Echt (NL); KLAASSEN, Paul, 6100 AA Echt (NL)
(74) Representative: DSM Intellectual Property

(56) References cited:
- WO-A1-2006/080822
- WO-A1-2012/136830
- WO-A1-2012/136832
- WO-A1-2013/153070
- WO-A1-2013/153074
- YANG E. ET AL: "Antimicrobial activity of bacteriocin-producing lactic acid bacteria isolated from cheeses and yogurts", AMB EXPRESS, vol. 2, no. 1, 10 September 2012 (2012-09-10), pages 1-12, XP002761383, DOI: 10.1186/2191-0855-2-48
- Ananou S. ET AL: "Biopreservation, an ecological approach to improve the safety and shelf-life of foods" In: A. Méndez-Vilas (Ed.): "Communicating Current Research and Educational Topics and Trends in Applied Microbiology", 2007, XP055396228, ISBN: 978-84-611-9423-0 * the whole document *

## Description

### Field of the invention

The present invention relates to a novel *Lactobacillus rhamnosus* strain which is suitable for providing an antimicrobial effect in dairy and in dairy based products. According to another aspect, the present invention relates to an antimicrobial, antifungal or anti-yeast composition. According to yet another aspect, the present invention relates to a food product. According to another aspect, the invention relates to a method for manufacturing a food product. According to another aspect, the present invention relates to the use of a *Lactobacillus rhamnosus* strain for providing an antifungal effect in silage or in dairy products.

### Background of the invention

Lactic acid bacteria are known for thousands of years for their role in the preparation of fermented foods, such as for instance dairy, meat and vegetable based products. During fermentation, lactic acid and other organic compounds are being produced by the lactic acid bacteria, thereby reducing the pH of the food product and consequently making it unfavourable to the growth of undesired micro-organisms, such as yeast(s), mould(s) and (pathogenic) bacteria. Without wishing to be bound by theory, examples of compounds that are being produced by lactic acid bacteria during fermentation have been described by e.g. Ananou et al (1), Delavenne et al (2) and Woraprayote et al (3).

Bioprotection, or biopreservation, is defined as the extension of shelf life and enhanced safety of foods by the use of natural or controlled microbiota and/or antimicrobial compounds (1). To this end, certain microbial ingredients, such as e.g. lactic acid bacteria, are being used in order to control the growth of undesired microorganisms such as yeasts, moulds and bacteria. Another example of such an effect was first identified by Alexander Fleming for a fungus having an inhibitory effect on the growth of bacteria. The result of this finding was the development of the medicament penicillin. Besides fungi, lactic acid bacteria are of particular interest for bioprotection since some may have antagonistic properties which make them useful as a bioprotectant.

Dairy products generally comprise living microorganisms such as lactic acid bacteria. However, fungi such as moulds and yeasts could grow abundantly in dairy products, even under cold conditions. Although the presence of lactic acid bacteria in dairy products is desired, there is a challenge to reduce the amount of yeasts and moulds.

Food grade chemical preservatives such as potassium sorbate and benzoate are common adequate measures to prevent the undesired growth of yeasts, moulds and bacteria. A drawback of these food grade chemical preservatives is that they are non-natural products having an 'E-number' and thus the products preserved with such a food grade chemical preservative could not have a clean label. This is undesirable in view of the increasing consumer's demand to natural products.

WO2013/153074 describes a *Lactobacillus rhamnosus* strain CHCC5366 and *Lactobacillus paracasei* strains CHCC12777 and CHCC14676. It is disclosed that the combination of the *Lactobacillus rhamnosus* strain with the *Lactobacillus paracasei* strain provides a synergistic effect in comparison with the strains alone.

WO2012/136830 describes an antimicrobial composition comprising at least one *Lactobacillus rhamnosus* strain and at least one *Lactobacillus paracasei* strain. A significant synergistic antimicrobial effect if both strains are combined is reported.

The commercial use of a combination of *Lactobacillus rhamnosus* and *Lactobacillus paracasei* is FreshQ®, which is a protective culture for fermented milk products available from Chr. Hansen, Denmark.

It is a drawback to use protective cultures consisting of combinations of different species because it increases the risk that this extra species introduces flavour to the food product. For the protective culture FreshQ® it is for example known that it introduces a creamy flavour to the fermented food product. This is a disadvantage because it limits the applications of the protective culture in for example non creamy applications like plain yogurt.

Therefore, there is an industrial need to develop a bioprotective solution to replace potassium sorbate wherein the bioprotective solution only increases the shelf-life stability without affecting other properties of the final product, such as e.g. flavour or pH.

### Summary of the invention

The objective of the present invention is the provision of novel strains of lactic acid bacteria with high efficacy as bioprotective agents, but no effect on flavour or on other attributes such as post-acidification.

This objective, amongst other objectives, is met by providing a *Lactobacillus rhamnosus* strain according to the appended claim 1.

More specifically, this objective, amongst other objectives, is met by providing a *Lactobacillus rhamnosus* strain deposited as CBS141584, or mutants derived therefrom, wherein said mutants have the same or improved antimicrobial, antifungal and/or anti-yeast properties as strain CBS141584.

According to another aspect, the present invention relates to an antimicrobial composition comprising the present *Lactobacillus rhamnosus* strain.

According to another aspect, the present invention relates to an antifungal composition comprising the present *Lactobacillus rhamnosus* strain.

According to yet another aspect, the present invention relates to a food product comprising the present *Lactobacillus rhamnosus* strain, the present antimicrobial composition or the present antifungal composition.

According to yet another aspect, the present invention relates to a method for manufacturing a food product comprising adding the present *Lactobacillus rhamnosus* strain, the present antimicrobial composition or the present antifungal during manufacture of the food product.

According to yet another aspect, the present invention relates to the use of the present *Lactobacillus rhamnosus* for providing an antifungal effect in dairy products or in silage.

### Detailed description of the invention

As used herein, the term "lactic acid bacteria" (LAB) or "lactic bacteria" refers to food-grade bacteria producing lactic acid as the major metabolic end-product of carbohydrate fermentation. These bacteria are related by their common metabolic and physiological characteristics and are usually Gram positive, low-GC, acid tolerant, non-sporulating, non-respiring, rod-shaped bacilli or cocci. During the fermentation stage, the consumption of sugars by these bacteria causes the formation of lactic acid and reduces the pH. These bacteria are thus responsible for the acidification and in some cases (e.g. in case of milk fermenttaions) for the texture of the fermented product. As used herein, the term "lactic acid bacteria" or "lactic bacteria" encompasses, but is not limited to, bacteria belonging to the genus of *Lactobacillus* spp., *Bifidobacterium* spp., *Streptococcus* spp., *Lactococcus* spp., such as *Lactobacillus delbruekii* subsp. *bulgaricus, Streptococcus thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.*

The term "milk" is intended to encompass milks from mammals, from plant sources or recombinant produced milk. Preferably, the milk is from a mammal source. Mammal sources of milk include, but are not limited to cow, sheep, goat, buffalo, camel, llama, mare and deer. In an embodiment, the milk is from a mammal selected from the group consisting of cow, sheep, goat, buffalo, camel, llama, mare and deer, and combinations thereof. Plant sources of milk include, but are not limited to, milk extracted from soy bean, pea, peanut, barley, rice, oat, quinoa, almond, cashew, coconut, hazelnut, hemp, sesame seed and sunflower seed. In addition, the term "milk" refers to not only whole milk, but also skim milk or any liquid component derived thereof.

As used in the present specification, the term "fermented milk product" refers to a product that has been fermented with lactic acid bacteria. Examples of lactic acid bacteria are *Streptococcus thermophilus* and *Lactobacillus delbruekii* subsp. *bulgaricus,* but also, optionally, other microorganisms such as for instance *Lactobacillus delbruekii* subsp. *lactis, Bifidobacterium animalis* subsp. *lactis, Lactococcus lactis, Lactobacillus acidophilus and Lactobacillus casei,* or any microorganism derived therefrom. The fermentation process increases the shelf-life of the product while enhancing and improving the digestibility of milk. Many different types of fermented milk products can be found in the world today. Examples are soured milk (e.g. buttermilk), sour cream and yogurt.

The term "starter culture" (also referred to as "starter") as used herein refers to a composition comprising one or more lactic acid bacteria, which are responsible for the acidification of the milk or milk base. Starter cultures compositions may be fresh (liquid), frozen or freeze-dried. Freeze dried cultures need to be regenerated before use. For the production of a fermented milk product, the starter cultures composition is usually added in an amount from 0.01 to 3%, preferably from 0.01 and 0.02 % by weight of the total amount of milk or milk base.

The term "mutant" should be understood as a strain derived from a strain of the invention, obtained by means of e.g. chemical mutagenesis, radiation, or genetic engineering. Mutants may even arise in a population that is not actively treated to obtain mutants, by means of errors during DNA replication (so-called "spontaneous" mutations) and are also included herein. Many methods are known in the art for obtaining mutants and methods for selecting mutants with desired properties are well known. The mutant has the same or improved antimicrobial, antifungal and/or anti-yeast properties as the strain of the invention deposited as CBS141584 from which it is derived.

The term "thermophile" or "thermophilic" herein refers to lactic acid bacteria that thrive well at temperatures above 41°C. The most useful thermophilic lactic acid bacteria include Lactobacillus spp. and Streptococcus ssp. Hence, a "thermophilic fermentation" herein refers to a fermentation that is being executed at a temperature above about 35°C, for example between about 35°C and about 45°C, such as e.g. at 42°C.

The term "mesophile" or "mesophilic" herein refers to lactic acid bacteria that thrive best at temperatures lower than 41°C, such as e.g. between about 15°C and about 40°C. Examples of mesophilic lactic acid bacteria with industrial relevance include for instance Lactococcus ssp. and Leuconostoc ssp. Hence, a "mesophilic fermentation" herein refers to a fermentation that is being done at a temperature between about 20° and 36°C, such as e.g. at 28°C.

The term "fermentation" herein refers to a metabolic process wherein sugar(s) are being converted into acids, gases, or alcohol. Fermentation occurs in many different cell types, such as e.g. yeasts and bacteria. Preferably, fermentation comprises the conversion of lactose into lactic acid.

"Undesired micro-organisms", "undesired contaminants" and "contaminants" herein refer to the occurrence of micro-organisms, such as bacteria, yeasts, moulds or a combination thereof, which bring about a negative perception of the food. The contaminant may be pathogenic, may have the ability to detoriate food products or, give rise to an unpleasant smell, taste or appearance of the food product. The strain of the invention is providing a solution in the prevention of the appearance and/or growth of such contaminants by inhibiting and/or preventing their growth upon entry in the dairy matrix, such as e.g. a yogurt or a sour cream product. The prevention of the growth of contaminants due to the action the present *Lactobacillus rhamnosus* strain can be expressed by e.g. a lower number of contaminant cell counts in a dairy product prepared with the present *Lactobacillus rhamnosus,* compared to a similar product, without the present *Lactobacillus rhamnosus* strain.

As used herein, the term antimicrobial, an antifungal or an anti-yeast composition means a composition suitable for providing an antimicrobial, an antifungal or an anti-yeast efficacy.

The present inventors have identified a novel *Lactobacillus rhamnosus* strain suitable for providing an antimicrobial effect. It was surprisingly found that this *Lactobacillus rhamnosus* strain provides an equal or even improved antimicrobial effect in view of the commonly used chemical preservative potassium sorbate. Therefore, in a preferred embodiment, the present *Lactobacillus rhamnosus* strain has an improved antimicrobial effect in a food product than potassium sorbate wherein the potassium sorbate is dosed in an amount of 0.05% (w/w) of the food product. Preferably, wherein the *Lactobacillus rhamnosus* strain is dosed in an amount of 0.001%(w/w) to 0.1%(w/w) of a medium or substrate, such as for example milk.

Further, the present inventors found that the present *Lactobacillus rhamnosus* strain is able to match or even improve the antimicrobial effect of a combination of another *Lactobacillus rhamnosus* strain and *Lactobacillus paracasei* strain such as FreshQ®. Furthermore, the present inventors found that the present *Lactobacillus rhamnosus* strain does not introduce a flavour effect to the food product. This enables the present *Lactobacillus rhamnosus* strain to be used in a broad range of food applications without altering the flavour profile of the food item. Therefore, in a preferred embodiment, the present *Lactobacillus rhamnosus* strain is suitable as bioprotectant without introducing a flavour effect to the food product the strain is added to.

The present *Lactobacillus rhamnosus* strain is deposited as CBS141584. Strain CBS141584 is deposited on July 5^{th}, 2016 at the Centraalbureau voor Schimmelcultures (Fungal Biodiversity Centre), Uppsalalaan 8, 3584 CT Utrecht, The Netherlands under the provisions of the Budapest Treaty. Mutants derived therefrom as used in the present context means *Lactobacillus rhamnosus* strains which are derived from CBS141584, or obtained from CBS141584, and may have mutations in comparison with *Lactobacillus rhamnosus* CBS141584 wherein the mutations do not alter the bioprotective phenotype of the derived *Lactobacillus rhamnosus* strain, i.e. wherein the mutant strain has the same or improved antimicrobial, antifungal and / or anti-yeast properties as the mother strain CBS141584. Preferably, the derived *Lactobacillus rhamnosus* strain is suitable for providing an antifungal effect and/or an anti-bacterial effect, as found for strain CBS141584. Preferably, the mutant derived from CBS141584 has more than 100% of the antimicrobial, antifungal, and / or anti-yeast effect compared with strain CBS141584 if compared under equal conditions.

Therefore, in a preferred embodiment, the present *Lactobacillus rhamnosus* strain is suitable for providing an antifungal effect and/or an anti-yeast effect, more preferably without introducing a flavour effect.

According to another aspect, the present invention relates to an antimicrobial, an antifungal and/or an anti-yeast composition comprising the present *Lactobacillus rhamnosus* strain. Preferably, the amount of the present *Lactobacillus rhamnosus* strain in the antimicrobial, antifungal and/or anti-yeast composition is sufficient to provide an antimicrobial, an antifungal or an anti-yeast effect. This enables the antimicrobial, antifungal or anti-yeast composition suitable to provide an antimicrobial, an antifungal or an anti-yeast effect.

In a preferred embodiment, the present antimicrobial, an antifungal and/or an anti-yeast composition further comprises a *Lactobacillus sakei* strain, preferably a *Lactobacillus sakei* strain as found in deposit CBS141161 or mutants derived therefrom. The present inventors identified that whereas *Lactobacillus sakei* is generally known for its anti-listeria activity, the addition of a *Lactobacillus sakei* strain to the present *Lactobacillus rhamnosus* strain improves the bioprotective activity against yeast and moulds. More specifically, the present inventors identified that *Lactobacillus sakei* strain CBS141161 alone does not provide bioprotective activity against yeast and molds, but the addition of strain CBS141161 to the present *Lactobacillus rhamnosus* strain further improves the bioprotective activity of the latter.

The present *Lactobacillus sakei* strain is preferably *Lactobacillus sakei* strain CBS141161. CBS141161 is deposited on March 16^{th}, 2016 at the Centraalbureau voor Schimmelcultures, Utrecht, the Netherlands.

The present antimicrobial, antifungal and/or anti-yeast composition typically comprises the present *Lactobacillus rhamnosus* strain in a concentrated form including frozen, dried or freeze-dried concentrates typically having a concentration of viable cells, which is in the range of 10⁴ to 10¹³cfu (colony forming units) per gram of the composition including at least 10⁴ cfu per gram of the composition, such as at least 10⁵ cfu/g, e. g. at least 10⁶ cfu/g, such as at least 10⁷ cfu/g, e.g. at least 10⁸cfu/g, such as at least 10⁹ cfu/g, e.g. at least 10¹⁰ cfu/g, such as at least 10¹¹ cfu/g. Thus, the antimicrobial, antifungal or anti-yeast composition of the invention is preferably present in a frozen, dried or freeze-dried form, e.g. as a Direct Vat Culture (DVC). However, as used herein the composition may also be a liquid that is obtained after suspension of the frozen, dried or freeze-dried cell concentrates in a liquid medium such as water, milk or PBS buffer. Where the composition of the invention is a suspension, the concentration of viable cells is in the range of 10⁴ to 10¹² cfu (colony forming units) per ml of the composition including at least 10⁴ cfu per gram of the composition, such as at least 10⁵ cfu/ml, e. g. at least 10⁶ cfu/ml, such as at least 10⁷ cfu/ml, e.g. at least 10⁸cfu/ml, such as at least 10⁹cfu/ml, e.g. at least 10¹⁰ cfu/ml, such as at least 10¹¹ cfu/ml.

The present antimicrobial, antifungal and/or anti-yeast composition typically comprises the present *Lactobacillus sakei* strain in a concentrated form including frozen, dried or freeze-dried concentrates typically having a concentration of viable cells, which is in the range of 10⁴ to 10¹³cfu (colony forming units) per gram of the composition including at least 10⁴ cfu per gram of the composition, such as at least 10⁵ cfu/g, e. g. at least 10⁶cfu/g, such as at least 10⁷ cfu/g, e.g. at least 10⁸cfu/g, such as at least 10⁹cfu/g, e.g. at least 10¹⁰ cfu/g, such as at least 10¹¹ cfu/g. Where the composition of the invention is a suspension, the concentration of viable cells is in the range of 10⁴ to 10¹² cfu (colony forming units) per ml of the composition including at least 10⁴ cfu per gram of the composition, such as at least 10⁵ cfu/ml, e. g. at least 10⁶ cfu/ml, such as at least 10⁷ cfu/ml, e.g. at least 10⁸cfu/ml, such as at least 10⁹ cfu/ml, e.g. at least 10¹⁰ cfu/ml, such as at least 10¹¹ cfu/ml.

The present antimicrobial, antifungal or anti-yeast composition may as further components contain cryoprotectants and/or conventional additives including nutrients such as yeast extracts, sugars and vitamins, e.g. vitamin A, C, D, K or vitamins of the vitamin B family. Suitable cryoprotectants that may be added to the composition of the invention are components that improve the cold tolerance of the microorganisms, such as mannitol, sorbitol, sodium tripolyphosphate, xylitol, glycerol, raffinose, maltodextrin, erythritol, threitol, trehalose, glucose, sucrose and fructose. Other additives may include, e.g., carbohydrates, flavors, minerals, enzymes (e.g. rennet, lactase and/or phospholipase).

More preferably, the present antimicrobial, antifungal or anti-yeast composition is packed. Preferably in a package which is suitable for shipment and/or storage of the present antimicrobial, antifungal or anti-yeast composition for at least 1 month, such as at least 3 months. Preferably the amount of antimicrobial, antifungal or anti-yeast composition in the package is at least 50 grams, such as at least 100 grams or 500 grams.

In a further aspect, the present invention relates to a food product comprising the present *Lactobacillus rhamnosus* strain of the invention, preferably with the present *Lactobacillus sakei* as described herein, or the present antimicrobial and/or antifungal composition.

The present invention relates to a food product comprising an amount of the present *Lactobacillus rhamnosus* strain of the invention, preferably with an amount of the present *Lactobacillus sakei* strain as described herein, which is effective for imparting antimicrobial properties to the food product. More preferably, wherein the presence of the present *Lactobacillus rhamnosus* strain and/or an amount of the present *Lactobacillus sakei* strain does not introduce a flavour to the food product.

In a further preferred embodiment, the present food product comprises the present *Lactobacillus rhamnosus* strain in an amount which is in the range of 10⁴ to 10¹²cfu (colony forming units) per gram of the food product including at least 10⁴ cfu per gram of the food product, such as at least 10⁵ cfu/g, e. g. at least 10⁶ cfu/g, such as at least 10⁷ cfu/g, e.g. at least 10⁸cfu/g, such as at least 10⁹ cfu/g, e.g. at least 10¹⁰ cfu/g, such as at least 10¹¹ cfu/g of the food product. More preferably, the present food product comprises the present *Lactobacillus rhamnosus* strain in an amount which is in the range of 10⁴ to 10¹²cfu (colony forming units) per cm² surface of the food product including at least 10⁴ cfu per cm² of the food product, such as at least 10⁵ cfu/cm², e. g. at least 10⁵ cfu/cm², such as at least 10⁷ cfu/cm², e.g. at least 10 ⁸cfu/cm², such as at least 10⁹ cfu/cm², e.g. at least 10¹⁰ cfu/cm², such as at least 10¹¹ cfu/ cm² surface of the food product.

In a further preferred embodiment, the present food product comprises the present *Lactobacillus sakei* strain in an amount which is in the range of 10⁴ to 10¹²cfu (colony forming units) per gram of the food product including at least 10⁴ cfu per gram of the food product, such as at least 10⁵ cfu/g, e. g. at least 10⁶ cfu/g, such as at least 10⁷ cfu/g, e.g. at least 10⁸cfu/g, such as at least 10⁹ cfu/g, e.g. at least 10¹⁰ cfu/g, such as at least 10¹¹ cfu/g of the food product. More preferably, the present food product comprises the present *Lactobacillus sakei* strain in an amount which is in the range of 10⁴ to 10¹²cfu (colony forming units) per cm² surface of the food product including at least 10⁴ cfu per cm² of the food product, such as at least 10⁵ cfu/cm², e. g. at least 10⁵ cfu/cm², such as at least 10⁷ cfu/cm², e.g. at least 10⁸cfu/cm², such as at least 10⁹cfu/cm², e.g. at least 10¹⁰cfu/ cm², such as at least 10¹¹ cfu/cm² surface of the food product.

Preferably, the present food product has a flavour profile which is comparable or indistinguishable from the food product, or from the same food product, which does not comprise the present *Lactobacillus rhamnosus* strain and/or the present *Lactobacillus sakei* strain. In other words, the present *Lactobacillus rhamnosus* strain and/or the present *Lactobacillus sakei* strain do not introduce a flavour to a food product to which the *Lactobacillus rhamnosus* strain and/or the present *Lactobacillus sakei* strain is added.

Preferably, the present food product in all aspects of the present invention is a dairy product. More preferably a fermented milk product, such as a mesophilic or thermophilic fermented milk product. Most preferably the present food product is sour cream or yogurt. Examples of a fermented dairy product of the present invention are various types of regular yoghurt, plain yogurt, low fat yoghurt, non fat yoghurt, tvarog, kefir, dahi, ymer, buttermilk, butter, sour cream and sour whipped cream. Other examples of fermented dairy product are cheese. For example fresh cheeses, unripened cheeses or curd cheeses. Alternatively, the fermented dairy product is a ripened cheese. Alternatively, the present food product is a dairy product, more preferably milk, whey, milk powder or whey powder.

According to yet another aspect, the present invention relates to a method for manufacturing a food product comprising adding a *Lactobacillus rhamnosus* strain of the invention, preferably with a *Lactobacillus sakei* strain as described herein, or the present antimicrobial composition, antifungal composition or anti-bacterial composition, during manufacture of the food product. Preferably, the present method comprises addition of the present *Lactobacillus rhamnosus* strain, preferably with a *Lactobacillus sakei* strain, or the present antimicrobial, antifungal or anti-yeast composition, to milk before fermentation of the milk. More preferably, the present *Lactobacillus rhamnosus* strain, preferably with a *Lactobacillus sakei* strain, or the present antimicrobial, antifungal or anti-yeast composition is added to the milk together with lactic acid bacteria used for fermentation of the milk. More preferably, the present method comprises a step of fermenting milk with lactic acid bacteria. Even more preferably, the present method comprises a step of fermenting milk with lactic acid bacteria in the presence of the present *Lactobacillus rhamnosus,* preferably with present *Lactobacillus sakei* strain, or in the presence of the present antimicrobial, antifungal or anti-yeast composition. The present inventors found that a high efficacy against yeast and moulds can be obtained if the present *Lactobacillus rhamnosus* strain is present during fermentation of the food product.

Thus, in a preferred embodiment, the method comprises one or more fermentation steps. Preferably, the method comprises fermenting a milk substrate with a starter culture comprising at least one strain of the genera selected from *Lactobacillus, Streptococcus, Lactococcus* and *Leuconostoc.* The present step of fermenting a milk substrate can be fermenting under mesophilic or under thermophilic conditions. More preferably, the present method is a method for manufacturing yogurt comprising adding a *Lactobacillus rhamnosus* strain of the invention, preferably with a *Lactobacillus sakei* strain as described herein, or the present antimicrobial composition, antifungal composition or anti-bacterial composition, during fermentation of milk with a starter culture comprising *Streptococcus thermophilus and Lactobacillus delbrueckii subsp. bulgaricus.* Alternatively, the present method is a method for manufacturing sour cream comprising adding a *Lactobacillus rhamnosus* strain of the invention, preferably with a *Lactobacillus sakei* strain as described herein, or the present antimicrobial composition, antifungal composition or anti-bacterial composition, during fermentation of milk with a starter culture comprising *Lactoccus lactis subsp. cremoris* and *Lactococcus lactis subsp. lactis.*

According to another aspect, the present invention relates to the use of the present *Lactobacillus rhamnosus* strain for providing an antimicrobial effect in dairy products, preferably for providing an antifungal effect and/or an anti-yeast effect in dairy products. More preferably wherein the dairy product is a fermented dairy product. More preferably a fermented dairy product such as various types of regular yoghurt, low fat yoghurt, non fat yoghurt, kefir, dahi, ymer, buttermilk, butter, sour cream and sour whipped cream. Other examples of a fermented dairy product wherein the present *Lactobacillus rhamnosus* strain can be used is cheese. For example fresh cheeses, unripened cheeses or curd cheeses. Alternatively, the fermented dairy product is a ripened cheese.

In a preferred embodiment, the present invention relates to to the use of the present *Lactobacillus rhamnosus* strain for providing an antimicrobial effect in silage, preferably for providing an antifungal effect and/or an anti-yeast effect in silage.

Further described is the use of the present *Lactobacillus sakei* strain for providing antifungal effect and/or an anti-yeast effect in dairy products. More preferably, described is the use of the present *Lactobacillus sakei* strain for enhancing the bioprotective efficacy of a *Lactobacillus rhamnosus* strain.

The invention is further illustrated in the non-limiting examples below. Reference is made to figure 1 to 12, showing:
- Figure 1:: Sour cream samples, deliberately contaminated with fungal spores, after 21, 28 and 42 days of storage 7°C. The added strain CBS141584 is indicated. In case of "Control", no biopreservative has been added, while PS contains 0.05% w/w potassium sorbate (chemical reference), added after the fermentation. FreshQ®1 is a market reference.
- Figure 2:: Sour cream samples, deliberately contaminated with fungal spores, after 7 and 14 days of storage at 21°C. The added strain CBS141584 is indicated. In case of "Control", no biopreservative has been added, while PS contains 0.05% w/w potassium sorbate (chemical reference), added after the fermentation. FreshQ®1 is a market reference.
- Figure 3:: Yeast cell counts (in CFU/ml) as a function of time (weeks) in yeast-contaminated sour cream samples, stored at 7°C. The lines of potassium sorbate, strain CBS141584 and FreshQ®1 are on top of each other.
- Figure 4:: Yeast cell counts (in CFU/ml) as a function of time (weeks) in yeast-contaminated sour cream samples, stored at 21°C. The lines of strain CBS141584 and potassium sorbate are on top of each other.
- Figure 5:: Sweet yogurt samples, deliberately contaminated with fungal spores, after 21, 28 and 42 days of storage at 7°C. The strain CBS141584 is indicated. In case of "Control", no biopreservative has been added, while PS contains 0.05% w/w potassium sorbate (chemical reference), added after the fermentation. FreshQ®2 is a market reference.
- Figure 6:: Sweet yogurt samples, deliberately contaminated with fungal spores, after 7 and 14 days of storage at 21°C. The added strain CBS141584 is indicated. In case of "Control", no biopreservative has been added, while PS contains 0.05% w/w potassium sorbate (chemical reference), added after the fermentation. FreshQ®2 is a market reference.
- Figure 7:: Yeast cell counts (in CFU/gram) as a function of time (days) in sweet yogurt samples contaminated with yeast cells and stored at 7°C. The lines of CBS141584, FreshQ®2 and potassium sorbate are on top of each other.
- Figure 8:: Brookfield viscosity (in cP) of the plain yogurt samples, FVV-231 as such and in combination with strain CBS141584.
- Figure: 9:Yeast (*R*. *muciloginosa)* cell counts (in CFU/g) as a function of time (days) in yeast-contaminated plain yogurt samples, stored at 7°C. The line of potassium sorbate is below the detection level. The combination CBS141161 and CBS141584 suppressed the growth of this yeast better than the other bioprotective cultures.
- Figure: 10:Plain yogurt samples, deliberately contaminated with fungal spores of P. *brevicompactum,* after 56 days of storage 21°C. From left to right: control, CBS141584 (middle) and the combination of CBS141161 and CBS141584 (right).

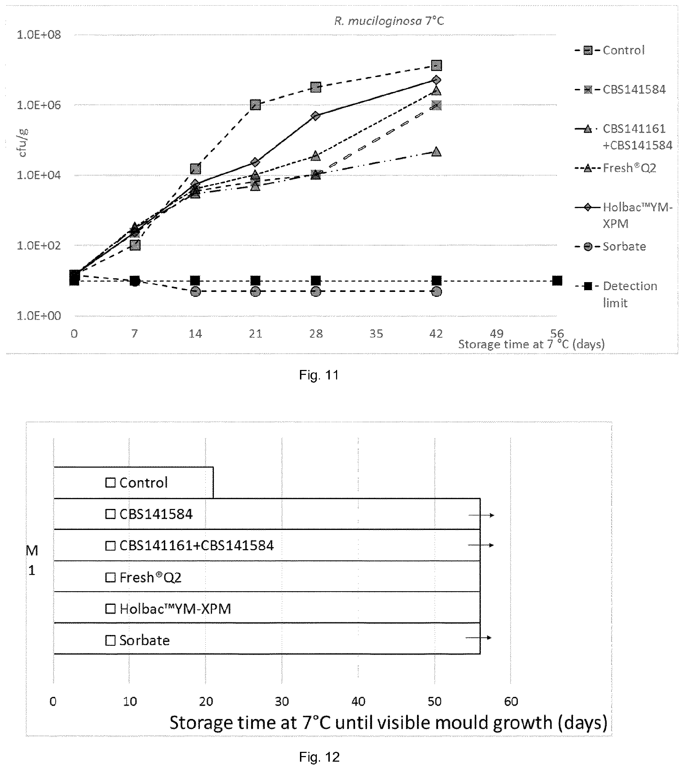

- Figure 11:: Yeast (*R*. *muciloginosa)* cell counts (in CFU/g) as a function of time (days) in yeast-contaminated sweet yogurt samples, stored at 7°C. The line of potassium sorbate is below the detection level. The combination CBS141161 and CBS141584 suppressed the growth of this yeast better than the other bioprotective cultures.
- Figure 12:: Sweet yogurt samples, deliberately contaminated with fungal spores of *P*. *brevicompactum,* after 56 days of storage 7°C. The control (no bioprotective culture included) exhibited contaminant growth after 21 days. The cultures Fresh®Q2 and Holdbac™YM-XPM showed mould growth after 56 days, while the yogurt samples indicated with the arrow (CBS141584, CBS141161 and CBS141584 and sorbate) are still without signs of mould growth.

### Examples

### Materials and methods

### Strains and products

**Table 1**

| **Strain or culture** | **Species composition** | **Obtainable from** |
|---|---|---|
| Delvo®Fresh FC-211 | *Lactococcus lactis subsp. cremoris Lactococcus lactis subsp. lactis* | DSM Food Specialties BV, Delft, the Netherlands |
| Delvo®Fresh YS-341 | *Streptococcus thermophilus Lactobacillus delbrueckii subsp. bulgaricus* | DSM Food Specialties BV, Delft, the Netherlands |
| Delvo®Fresh YS-131 | *Streptococcus thermophilus Lactobacillus delbrueckii subsp. bulgaricus* | DSM Food Specialties BV, Delft, the Netherlands |
| CBS141584 | *Lactobacillus rhamnosus* | CBS141584 is deposited on July 5^{th}, 2016 at the Centraalbureau voor Schimmelcultures, Utrecht, the Netherlands |
| CBS141161 | *Lactobacillus sakei* | CBS141161 is deposited on March 16^{th} 2016 at the Centraalbureau voor Schimmelcultures, Utrecht, the Netherlands |
| FreshQ®1 | *Lactococcus rhamnosus Lactobacillus paracasei* | Chr. Hansen, Hoersholm, Denmark |
| FreshQ®2 | *Lactobacillus rhamnosus* | Chr. Hansen, Hoersholm, Denmark |
| DELVO® YOG FVV-231 | *Streptococcus thermophilus Lactobacillus delbrueckii subsp. bulgaricus* | DSM Food Specialties BV, Delft, the Netherlands |
| ATCC34905 | *P. roqueforti* | ATCC (www.atcc.org) |
| ATCC18110 | *Debaryomyces hansenii* | ATCC (www.atcc.org) |
| Contaminant isolated from yoghurt | *Rhodotorula mucilaginosa* | Standard yogurt product |
| Wide spread dairy spoilage organism, in this case isolated from 9% creme fraiche | *Penicillium brevicompactum* | Standard Creme fraiche product |
| Well-known yogurt contaminant; isolated from yogurt | *Aspergillus flavus* | Standard yogurt product |

### Sour cream fermentation

Sour cream (SC) was manufactured according to a standard protocol. Specifically, for each run, about 5.5 liters of SC were produced per vat, with about 30% fat and about 2.4% protein. Per kilogram SC substrate, about 252 gram full fat milk was used and about 748 grams of cream. The SC substrate was pasteurized for 6 minutes at 92°C. In line homogenization takes place in the heating part of the pasteurizer at 60°C, in two stages of about 50 and 30 bar. Subsequently, the SC substrate was cooled down to 29°C.

Culture combinations were added according to the scheme below:

**Table 2**

| **Variation** | **Cultures added (inoculation rate)** |
|---|---|
| Delvo®Fresh FC-211 | FC-211 (0.01% w/w) |
| Delvo®Fresh FC-211 + CBS141584 | FC-211 (0.01% w/w) |
| | CBS141584 (0.01% w/w) |
| Delvo®Fresh FC-211 + FreshQ®1 | Delvo®Fresh FC-211 (0.01% w/w) |
| | FreshQ®1 (100U/1000 liter) |
| Delvo®Fresh FC-211 + 0.05% w/w potassium sorbate (PS) | FC-211 (0.01% w/w) |

An inoculation rate of 0.01% CBS141584 (and also CBS141161) corresponds to approximately 1.0E+6 CFU/ml at the start of the fermentation.

The dosage of FreshQ®1 was according to the instructions of the supplier.

After addition of the cultures, two portions of 200 ml were withdrawn for CINAC analysis (AMS France, Frepillion, France) allowing continuous monitoring of the fermentation activity by simultaneous measurement of pH and temperature in time, at 28°C. The remaining 5 liters of SC substrate were incubated in a water bath (28°C) until a pH of 4.6 was reached.

PS was added (in the variation indicated above) after the fermentation as a powder and was mixed by hand, prior to smoothening.

The product was smoothened by pumping the SC through two sieves (pore size 750 µm followed by 350 µm) whilst cooling with tap water. The SC was then filled out into suitable containers; some cups were deliberately contaminated for challenge tests and the cups were stored at 7°C and 21°C (for details, see section "Sour cream challenge tests").

### Sweet yogurt fermentation

Sweet yogurt was manufactured according to a standard protocol. Full fat milk was used. Specifically, for each run, about 5.5 liters of yogurt were produced per vat, with about 3.4% fat, about 3.4% protein and 8% sucrose.

The milk substrate was pasteurized for 6 minutes at 92°C. Subsequently, the milk substrate was cooled down to 43°C.

Culture combinations were added according to the scheme below:

**Table 3**

| **Variation** | **Cultures added (inoculation rate)** |
|---|---|
| Delvo®Fresh YS-341 | YS-341 (0.02% w/w) |
| Delvo®Fresh YS-341 + CBS141584 | YS-341 (0.02% w/w) |
| | CBS141584 (0.01% w/w) |
| Delvo®Fresh YS-341 + FreshQ®2 | Delvo®Fresh YS-341 (0.02% w/w) |
| | FreshQ®2 (100U/1000 liter) |
| FC-211 + 0.05% w/w potassium sorbate (PS) | YS-341 (0.02% w/w) |

The dosage of FreshQ®2 was according to the instructions of the supplier.

After addition of the cultures, two portions of 200 ml were withdrawn for CINAC analysis (AMS France, Frepillion, France) allowing continuous monitoring of the fermentation activity by simultaneous measurement of pH and temperature in time, at 42°C. The remaining 5 liters of milk substrate were incubated in a water bath (42°C) until a pH of 4.6 was reached.

PS was added (in the variation indicated above) after the fermentation as a powder and was mixed by hand, prior to smoothening.

The product was smoothened by pumping the yogurt through two sieves (pore size 750 □m followed by 350 µm) whilst cooling with tap water. The yogurt was then filled out into suitable containers; some cups were deliberately contaminated for challenge test and the cups were stored at 7°C and 21°C (for details, see section "Sweet yogurt challenge tests").

### Plain yogurt fermentation

Plain yogurt was manufactured according to a standard protocol. Specifically, for each run, about 5.0 liters of yogurt were produced per vat, with about 3.4% fat and about 4.2% protein. To this end, per kg of milk substrate, about 935 grams of full fat milk, 40 grams of skimmed milk and 25 grams of skimmed milk protein (SMP) were mixed.

The milk substrate was pasteurized for 6 minutes at 92°C. In line homogenization takes place in the heating part of the pasteurizer at 60°C, in two stages of 80 and 40 bar. Subsequently, the milk substrate was cooled down to 43°C.

Culture combinations were added according to the scheme below:

**Table 4**

| **Variation** | **Cultures added (inoculation rate)** |
|---|---|
| DELVO® YOG FVV-231 | DELVO® YOG FVV-231 (0.02% w/w) |
| DELVO® YOG FVV-231 + CBS141584 | DELVO® YOG FVV-231 (0.02% w/w) CBS141584 (0.01% w/w) |

After addition of the cultures, two portions of 200 ml were withdrawn for CINAC analysis (AMS France, Frepillion, France) allowing continuous monitoring of the fermentation activity by simultaneous measurement of pH and temperature in time, at 42°C. The remaining 4.5 liters of milk substrate were incubated in a water bath (42°C) until a pH of 4.6 was reached.

The product was smoothened by pumping the yogurt through two sieves (pore size 750 µm followed by 350 µm) whilst cooling with tap water. The yogurt was then filled out into suitable containers and stored at 7°C.

### Challenge tests

The fermented milk products of each of the variations described above were smoothened and split in three parts. One part was not contaminated, one part was contaminated with mould spores and one part was contaminated with yeast cells. The contaminants were added to the fermented milk products at about 100 mould spores per cup (*P. roqueforti* ATCC34905) or 50 yeast cells per milliliter (*Debaryomyces hansenii* ATCC18110). Both species are well-known dairy contaminants in the industry.

A mild homogenization was applied in order to mix the yeast cells through the fermented milk products after the yeast addition; the fungal spores were pipetted on top of the FMPs after filling the cups (about 125 grams per cup) and the tubes (about 20 grams per tube).

The cups and tubes were capped with appropriate lids and stored at 7°C and room temperature (21-22°C). Cups were inspected and photographed once a week for mould or yeast growth on the surface of the products, up to 8 weeks. Yeast counts were done by serial dilution of the samples and plating on OGY-agar (Sigma Aldrich).

### Brookfield viscosity analysis

Viscosity measurement were performed using a Brookfield RVDVII+ Viscometer, which allows viscosity measurement on an undisturbed product (directly in the pot). The Brookfield Viscometer determines viscosity by measuring the force required to turn the spindle into the product at a given rate. The Helipath system with a T-C spindle was used as it is designed for non-flowing thixotropic material (gels, cream). It slowly lowers or raises a rotating T-bar spindle into the sample so that not always the same region of the sample is sheared (helical path). Thus, the viscometer measures constantly the viscosity in fresh material, and is thus thought to be the most suitable for measuring stirred yogurt viscosity. A speed of 30 rpm was used for 31 measuring points, at an interval of 3 sec. The average of the values between 60 and 90 seconds are reported.

### Example 1

### Sour cream mould challenge test

Sour cream was produced as described in the Materials and Methods section. Subsequently, the challenge test was initiated as described above.

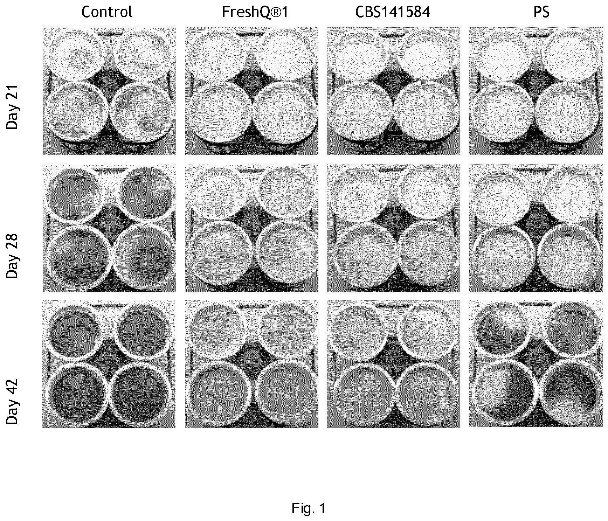

There was no visible fungal growth in all the cups stored at 7°C, in the first two weeks. In week 3 however, in the cups of the control, mould appeared on the surface of the sour cream samples, which grew out to a thick fungal carpet in the following weeks (see Figure 1). In the cups with FreshQ®1, CBS141584 and PS, fungal outgrowth was slowed down to a large extent compared to the control sample. However, fungal growth was slowed down best in the samples containing CBS141584. After four weeks (28 days), the spots where the matrix was inoculated with the mould spores were clearly disturbed, but no normal fungal growth was observed in case of CBS141584, but sporulated fungal colonies appeared in the sample containing FreshQ®1. This remained to be so until day 42; the control was fully overgrown by then, and also in the chemically preserved reference (PS) and the sample FreshQ®1, mould growth was evident, but still lagging behind in the sample containing CBS141584.

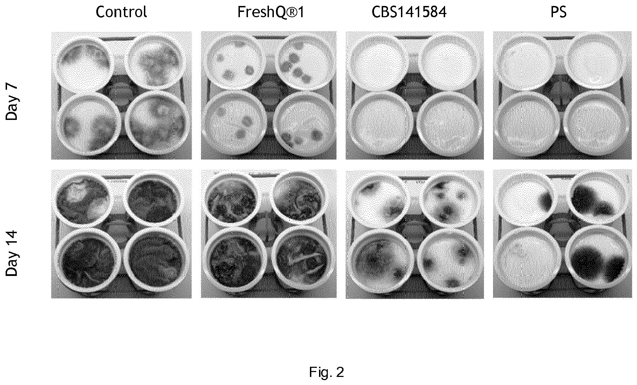

The same picture emerged during storage at 21°C, but fungal outgrowth was faster at this higher temperature, as expected (Figure 2). In the control, fungal growth was already visible after 1 week of incubation; after 2 weeks, the cups were fully overgrown (Figure 2). After one week of incubation, no fungal colonies appeared on the samples containing either strain CBS141584 or the chemical preservative PS, while fungal colonies emerged in the sample containing FreshQ®1 and even more severe in the control (no preservative added). After two weeks of incubation, the fungal outgrowth was more severe in case of the chemically preserved sour cream sample (PS) compared to the sour cream sample fermented in the presence of CBS141584. Also in the samples containing FreshQ®1 the fungal contamination was grown to a larger extent compared to CBS141584, indicating that the latter, being a single strain of *Lactobacillus rhamnosus,* provided a higher degree of biopreservation compared to FreshQ®1, which is a mixture of a strain of *Lactobacillus rhamnosus* and a strain of *Lactobacillus paracasei,* (Figure 2).

These results show that the bio-preservative action of CBS141584 is better than both the chemical reference PS and FreshQ®1 and slows down mould growth to a larger extent. It should be noted that the amount of spores spotted on the surface of the product is extremely high and not representative for typical industrial contamination levels.

### Example 2

### Sour cream yeast challenge test

Sour cream was produced as described in the Materials and Methods section. Subsequently, the challenge test was initiated as described above.

During storage at 7°C, no visible growth occurred for two weeks. After three weeks, colonies emerged at the surface of the sour cream control sample, but not in the cases in which FreshQ®1, strain CBS141584, and the chemical reference potassium sorbate (PS) were added. This situation remained like that until the end of the experiment, i.e. for eight weeks.

During storage at 21°C, no (yeast) contaminants were visible in the cups where CBS141584 and PS were added. The control however was overgrown by the yeast. Also in the case of FreshQ®1, some colonies emerged at the surface of the sour cream sample.

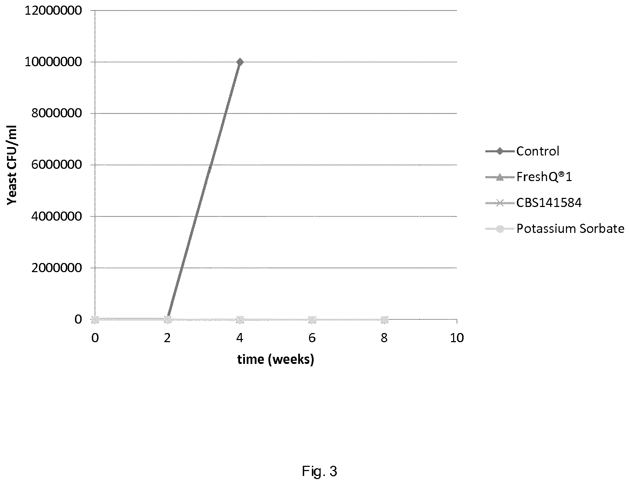

The cell counts indicated that at 7°C, yeast growth was abundant in the cups of the control (no adjunct during the fermentation). Yeast cell counts were lower in the other samples, i.e. FreshQ®1, CBS141584 and in the PS sample, which all perform equal (Figure 3).

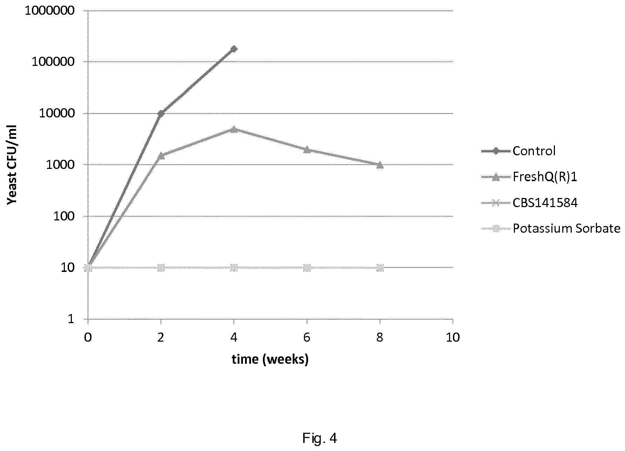

At 21°C, a similar picture emerged (see Figure 4); no yeast growth in case of the CBS141584 and PS samples, but fast and abundant yeast growth in case of the control (no adjunct biopreservative culture during the fermentation). The FreshQ®1 however, allowed for some yeast growth up to four weeks; from that point on, the yeast did not grow anymore. These results show that CBS141584, being a single strain of *Lactobacillus rhamnosus,* provided a higher degree of biopreservation compared to FreshQ®1, which is a mixture of a strain of *Lactobacillus rhamnosus* and a strain of *Lactobacillus paracasei,.*

### Example 3

### Sweet yogurt mould challenge test

Sweet yogurt was produced as described in the Materials and Methods section. Subsequently, the challenge test was initiated as described above.

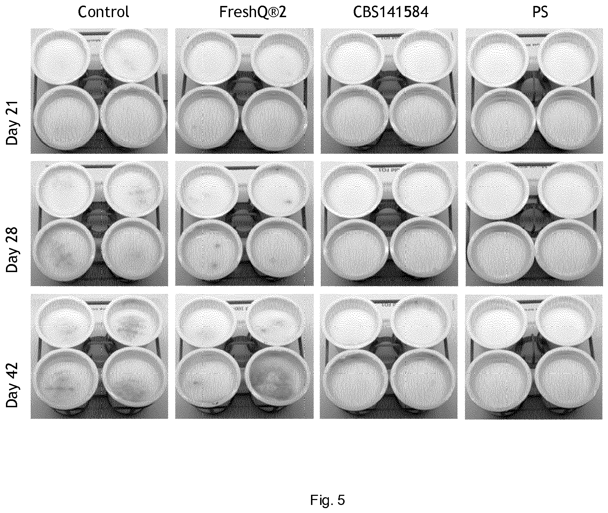

In the cups contaminated with fungal spores, no mould growth was observed during storage at 7°C for two weeks. In week 3 (21 days) and even more in week 4 (28 days), mould growth was evident in the control, as shown in Figure 5, but not in the cups filled with product produced with either CBS141584 as a biopreservative adjunct, or to which potassium sorbate (PS) was added. In the commercal reference FreshQ®2 however, mould growth was evident, as shown in Figure 5. After 6 weeks of incubation at 7°C (42 days, Figure5), it is even more evident that CBS141584 outperforms the control and FreshQ®2 and is comparable to the chemical reference potassium sorbate (PS).

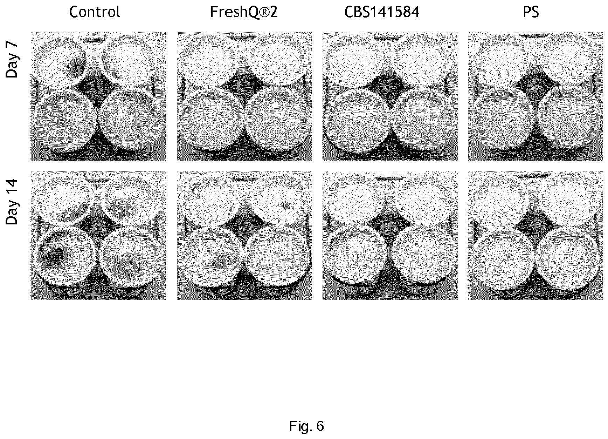

The results obtained during storage at 21°C were essentially the same, although fungal growth commenced earlier and cups were overgrown with a fungal blanket at a higher rate. Also in this case, CBS141584 suppressed fungal growth to the same extent as PS did (Figure 6) until 14 days of incubation and outperformed FreshQ®2 (Figure 6) since in the latter fungal growth was evident.

### Example 4

### Sweet yogurt yeast challenge test

Sweet yogurt was produced as described in the Materials and Methods section. Subsequently, the challenge test was initiated as described above.

At both 7°C and 21°C, the weekly visual inspection of the cups indicated that addition of CBS141584 as a biopreservative adjunct culture, or 0.05% w/w PS as a chemical preservative, inhibit yeast growth.

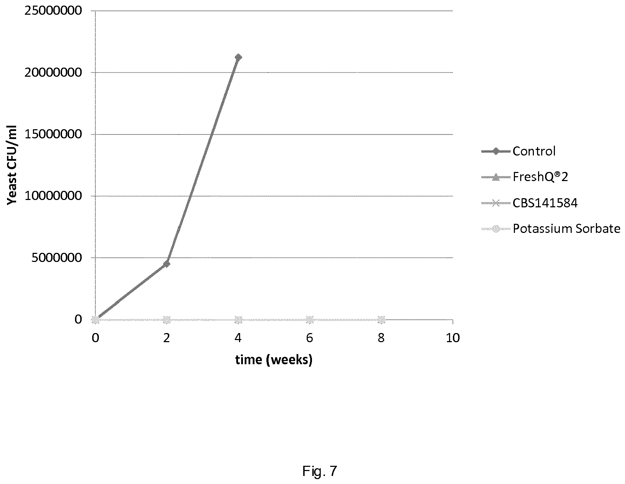

This was backed by the results of the cell counts; as an example, the results are shown of the experiment performed at 7°C (Figure 7). Clearly, growth was rapid and abundant in case of the control (no adjunct bio-preservative culture). In case of FreshQ®2, CBS141584 and PS, the cell counts remained low or even below detection level, demonstrating the bio-protective effect of CBS141584 under these conditions, being on par with the chemical reference PS and FreshQ®2.

At 21 °C, the results were essentially the same compared to the results described above for storage at 7°C.

### Example 5

### Effect of CBS141584 on post-acidifcation, viscosity and sensory analysis of plain yogurt

### Post-acidification

The post-acidification during storage at 5°C was followed in time. The results are shown the table below.

### Development of the pH during storage of the plain yogurt samples at 5°C

**Table 5**

| Culture | pH Day 1 | pH Day 7 | pH Day 14 | pH Day 28 |
|---|---|---|---|---|
| FVV-231 | 4.60 | 4.45 | 4.44 | 4.39 |
| FVV-231 + CBS141584 | 4.57 | 4.42 | 4.40 | 4.34 |

The difference between the two samples on day 1 is limited, about 0.03 pH units, and this difference changed only slightly in time, if the differences are significant. In both samples, the pH dropped about 0.2 pH units in 28 days (4 weeks).

### Brookfield viscosity analysis

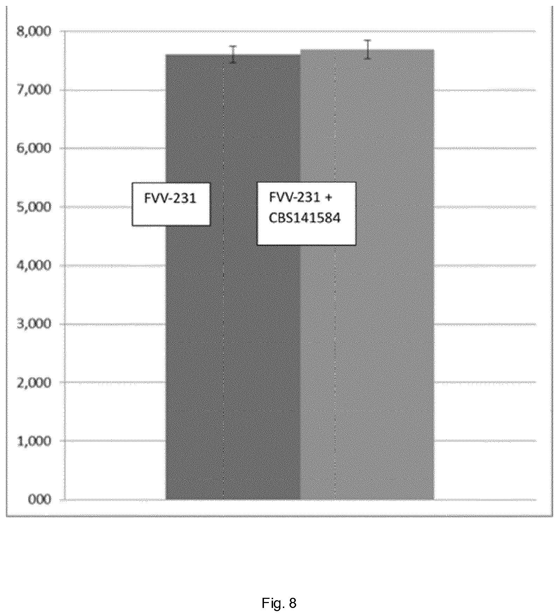

The viscosity of the plain yogurt samples was determined by Brookfield analysis. The results are depicted in Figure 8.
The Brookfield values are virtually identical for both samples: 7598 cP in case of FVV-231 and 7685 cP in case of FVV-231 + CBS141584. In both cases, the relative standard deviation is about 2%.

### Sensory analysis

The products (plain yogurt prepared with FVV-231 and plain yogurt prepared with FVV-231 and CBS141584 were assessed by an sensory panel by means of an R-index difference test. The panelists were asked if the two products differed from each other and how sure they were about their answer. A blind control was given as well.

In summary, the results showed that no statistically significant difference was found between the two products, which means that the strain CBS141584 can be used in combination with the FVV-231 culture without affecting the sensory perception of the final product.

### Example 6

### Challenge tests in yogurt prepared with a combination of bioprotective Lactobacillus strains

The bioprotective effect of a combination of two *Lactobacillus* strains was tested in a number of different FMP-matrices. To this end, plain yogurt (using starter culture YS-131) and sweet yogurt (starter culture YS-341) were prepared according to the protocols in the Materials and Methods section.

Plain yogurt was prepared as such, but also with adjunct bioprotective cultures:
a) Strain CBS141584 (0.01% w/w),
b) A combination of strain CBS141161 (0.0033 % w/w) and CBS141584 (0.0067% w/w),
c) Fresh®Q2 (100U/1000L; according to the instructions of the manufacturer),
d) Holdbac™YM-XPM (10DCU/1000L).

An additional reference was created by adding 0.05% (w/w) potassium sorbate to the product
prepared without a bioprotective adjunct.

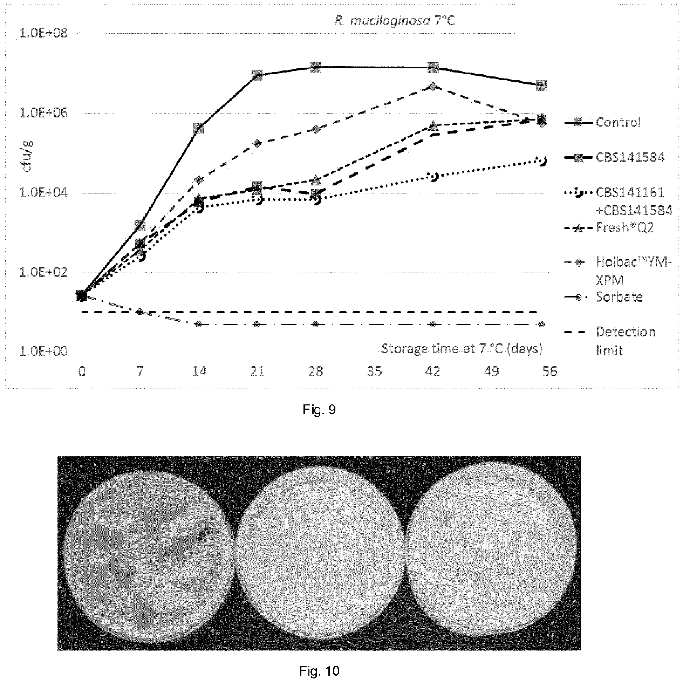

Subsequently, a challenge test was initiated, using several different contaminants. Amongst these contaminants were *R. mucilaginosa* and *P. brevicompactum* (see Table 1). The results are shown in Figure 8 (cell counts *R. muciloginosa*) and Figure 9 (storage time until visible mold growth, in days).

The results clearly showed that the cell counts of *R. muciloginosa* (Figure 8) are lowest in case a combination of CBS141161 and CBS141584 was used. The cell counts remained well below the level of 100.000 CFU/gram, even after 56 days at 7°C; yeasty and fermented off-flavors and gassy appearance are often detected when yeasts grow to 100.000-1.000.000 CFU/g (4). In case of the other biopreservative adjuncts, yeast growth was more prominent and often appeared to cross the level of 100.000 CFU/gram earlier in time compared to the sample containing CBS141584 and CBS141161.

*L. sakei* is generally known for its anti-listerial activity. We have previously seen that strain CBS141161 by itself does not provide bioprotective activity against yeast and molds. We surprisingly have now found that the addition of CBS141161 to strain CBS141584 further improves the bioprotective activity of the latter.

Figure 10 shows that the control (no biopreservative adjunct added) is completely overgrown by the contaminant, *P. brevicompactum.* The contamination was already visible after 5 days of incubation at 21°C. In case of CBS141584 only (middle panel), fungal growth was visible after 56 days, while the mixed adjunct culture (CBS141161 and CBS141584) was still "clean", i.e. no visible growth of the spiked contaminant.

Sweet yogurt was prepared as such, but also with adjunct bioprotective cultures:
a) Strain CBS141584 (0.01% w/w),
b) A combination of strain CBS141161 (0.0033 % w/w) and CBS141584 (0.0067% w/w),
c) Fresh®Q2 (100U/1000L),
d) Holdbac™YM-XPM (10DCU/1000L).

An additional reference was created by adding 0.05% (w/w) potassium sorbate to the product
prepared without a bioprotective adjunct.

Subsequently, a challenge test was initiated, using several different contaminants. Amongst these contaminants were *R. mucilaginosa* and *P. brevicompactum* (see Table 1). The results are shown in Figure 11 (cell counts *R. muciloginosa*) and Figure 12 (storage time until visible mold growth, in days).

The results clearly showed that the cell counts of *R. muciloginosa* (Figure 10) are lowest in case a combination of CBS141161 and CBS141584 was used. The cell counts remained well below the level of 100.000 CFU/gram, even after 42 days at 7°C; yeasty and fermented off-flavors and gassy appearance are often detected when yeasts grow to 100.000-1.000.000 CFU/g (4). In case of the other biopreservative adjuncts, yeast growth was more prominent and often appeared to cross the level of 100.000 CFU/gram earlier in time compared to the sample containing CBS141584 and CBS141161.

*L. sakei* is generally known for its anti-listerial activity. We have previously seen that strain CBS141161 by itself does not provide bioprotective activity against yeast and molds. We surprisingly have now found that the addition of CBS141161 to strain CBS141584 further improves (has a synergistic effect on) the bioprotective activity of the latter by an unknown mechanism.

Figure 12 shows that the control (no biopreservative adjunct added) exhibited growth of the contaminant, *P. brevicompactum,* after 21 days of incubation at 7°C. In case of CBS141584 only, fungal growth was not visible after 56 days, and also not in the case of the mixed adjunct culture (CBS141161 and CBS141584), the yogurt was still "clean", i.e. no visible growth of the spiked contaminant, like the chemical reference sorbate. In case Fresh®Q2 and Holdbac™YM-XPM were used as biopreservative adjuncts, growth of a yellowish mold was visible.

In conclusion, it is shown that in two different matrices, i.e. plain yogurt and sweet yogurt, the bioprotective effect of CBS141584, in combination with CBS141161 or as such, provided a higher bioprotective activity compared to Fresh®Q2 and Holdbac™YM-XPM against certain contaminants.

### Example 7

### Effect of bioprotective cultures on sensory evaluation of sour cream

Sour cream was produced at 5 liter scale, as described in the Materials and Methods section. The following six different products were made:

**Table 6**

| **Product #** | **Starter culture** | **Bioprotective adjunct** |
|---|---|---|
| 1 | Delvo®Fresh FC-211 | None |
| 2 | Delvo®Fresh FC-211 | CBS141161 (0.01% w/w) |
| 3 | Delvo®Fresh FC-211 | CBS141161 (0.0033% w/w) + CBS141584 (0.0067% w/w) |
| 4 | Delvo®Fresh FC-211 | CBS141584 (0.01% w/w) |
| 5 | Delvo®Fresh FC-211 | Fresh®Q1 (100U/1000L) |
| 6 | Delvo®Fresh FC-211 | 0.05% potassium sorbate (added after the fermentation) |

The products were evaluated on several attributes by ten panelists in duplicate, by means of QDA (Qualitative Data Analysis). The products were assessed at two time points: after one week of storage in the refrigerator, but also after four weeks after storage in the refrigerator.

The mean score of each product per attribute is given in Table 7 (week 1) and Table 8 (week 4 after production). The results of the ANOVA test, in order to assess whether the differences between the values are statistically different, are indicated in the table by letters. If two products have a different letter for a certain attribute (for example a 'b' instead of a 'a'), it means that the panel perceived a statistically significant difference between the products.

The abbreviations in the sensory tables behind the attribute designate the following: str = structure, od = odour, mf = mouthfeel, fl = flavour, at = aftertaste and af = afterfeel.

**Table 7**

| | FC211 control | | FC211 CBS141161 | | FC211 CBS141161 CBS141584 | | FC211 CBS141584 | | FC211 FreshQ1 | | FC211 Sorbate | | p-Value |
|---|---|---|---|---|---|---|---|---|---|---|---|---|---|
| syneresis-ap | 15 | | 17 | | 16 | | 10 | | 14 | | 11 | | 0.208 |
| thickness-str | 51 | | 57 | | 57 | | 56 | | 62 | | 56 | | 0.06 |
| smoothness-str | 58 | | 56 | | 55 | | 53 | | 53 | | 51 | | 0.167 |
| **shininess-str** | 69 | a | 69 | a | 65 | ab | 66 | a | 64 | ab | **59** | **b** | ** |
| intensity-od | 54 | | 53 | | 52 | | 55 | | 51 | | 52 | | 0.588 |
| sour-od | 30 | | 30 | | 25 | | 26 | | 22 | | 22 | | 0.053 |
| firmness-mf | 43 | | 44 | | 45 | | 48 | | 47 | | 45 | | 0.735 |
| **viscosity-mf** | 55 | b | 58 | b | 59 | ab | 59 | b | **64** | **a** | 58 | b | * |
| slipperiness-mf | 80 | | 79 | | 81 | | 79 | | 79 | | 80 | | 0.942 |
| creaminess-mf | 54 | | 56 | | 54 | | 58 | | 51 | | 55 | | 0.084 |
| melting-mf | 62 | | 62 | | 60 | | 62 | | 60 | | 64 | | 0.754 |
| intensity-fl | 58 | | 58 | | 57 | | 58 | | 59 | | 59 | | 0.955 |
| **sour-fl** | 44 | a | 45 | a | 44 | a | 41 | a | 46 | a | **36** | **b** | ** |
| cream-fl | 39 | | 42 | | 40 | | 42 | | 36 | | 39 | | 0.753 |
| sweet-fl | 23 | | 26 | | 25 | | 26 | | 25 | | 28 | | 0.091 |
| intensity-at | 47 | | 46 | | 46 | | 47 | | 48 | | 46 | | 0.983 |
| astringent-af | 21 | | 25 | | 25 | | 23 | | 25 | | 20 | | 0.147 |
| fat coating-af | 34 | | 31 | | 34 | | 35 | | 32 | | 37 | | 0.732 |

**Table 8**

| | FC211 control | | FC211 CBS141161 | | FC211 CBS141161 CBS141584 | | FC211 CBS141584 | | FC211 FreshQ1 | | FC211 Sorbate | | p-Value |
|---|---|---|---|---|---|---|---|---|---|---|---|---|---|
| syneresis-ap | 11 | | 9 | | 8 | | 9 | | 10 | | 8 | | 0.347 |
| **thickness-str** | 55 | bc | 59 | ab | 59 | ab | 62 | a | 62 | a | **52** | **c** | *** |
| smoothness-str | 61 | | 59 | | 59 | | 58 | | 56 | | 58 | | 0.598 |
| shininess-str | 63 | | 67 | | 61 | | 62 | | 59 | | 63 | | 0.216 |
| intensity-od | 48 | | 55 | | 53 | | 55 | | 56 | | 51 | | 0.065 |
| sour-od | 22 | | 28 | | 26 | | 27 | | 25 | | 24 | | 0.748 |
| firmness-mf | 41 | | 43 | | 43 | | 45 | | 43 | | 42 | | 0.722 |
| viscosity-mf | 60 | | 60 | | 59 | | 62 | | 61 | | 55 | | 0.155 |
| slipperiness-mf | 78 | | 78 | | 77 | | 79 | | 77 | | 78 | | 0.733 |
| creaminess-mf | 58 | | 58 | | 58 | | 57 | | 54 | | 58 | | 0.57 |
| melting-mf | 59 | | 60 | | 61 | | 55 | | 60 | | 62 | | 0.279 |
| **intensity-fl** | 59 | bc | 61 | ab | 60 | bc | 61 | b | **65** | **a** | **57** | **c** | ** |
| **sour-fl** | 35 | c | 47 | b | 44 | b | 41 | b | **53** | **a** | 34 | c | *** |
| cream-fl | 38 | | 37 | | 36 | | 37 | | 29 | | 37 | | 0.217 |
| **sweet-fl** | 25 | a | 21 | abc | 20 | bc | 21 | abc | **18** | **c** | 23 | ab | ** |
| intensity-at | 46 | | 51 | | 49 | | 48 | | 50 | | 50 | | 0.525 |
| **astringent-af** | 23 | b | 24 | b | 25 | ab | 24 | b | **29** | **a** | 22 | b | * |
| fat coating-af | 33 | | 32 | | 30 | | 33 | | 30 | | 34 | | 0.716 |

The results indicated that after one week (Table 7), there were not many significant differences between the different products, per attribute.

With respect to shininess at week 1, the product containing 0.05% sorbate (indicated by "b") differs from the control (no bioprotective adjunct), the product containing CBS141161 and the product containing CBS141584 (all indicated by "a"), however, all the above were not statistically different from the products prepared with the bioprotective adjuncts Fresh®Q1 and the mix of CBS141161 and CBS141584.

Likewise, the addition of the bioprotective adjunct Fresh®Q1 resulted in a significant difference in the attribute "viscosity-mf" (Table 7; "a") compared to most products.

The sensorial assessment after four weeks of storage in the refrigerator exhibited a statistically different value for Fresh®Q1, which appeared to be much more sour than the other products (Table 8). This indicated that post-acidification is more severe in this product compared to the other products. In line with this, the same product was perceived as the least sweet. Also, the product containing Fresh®Q1 was perceived as the most astringent.

In general, it can be concluded that the products containing CBS141161 and/or CBS141584 hardly differ from the control. From the results of week one, the odd product is the product with sorbate added, while at four weeks the Fresh®Q1 was the most deviating product.

### References

(1) Ananou S, Maqueda M, Martinez-Bueno M and Valdivia E (2007). Biopreservation, an ecological approach to improve the safety and shelf-life of foods. In Mendez-Vilas A (Ed.) Communicating Current Research and Educational Topics and trends in Applied Microbiology. Formatex. P. 475-486
(2) Delavenne et al. Biodiversity of antifungal lactic acid bacteria isolated from raw milk samples from cow, ewe and goat over one-year period. International Journal of Food Microbiology 155 (2012) 185-190
(3) Woraprayote et al. Bacteriocins from lactic acid bacteria and their applications in meat and meat products. Meat Science 120 (2016) 118-132

## Claims

1. A *Lactobacillus rhamnosus* strain deposited as CBS141584, or mutants derived therefrom, wherein said mutants have the same or improved antimicrobial, antifungal and/or anti-yeast properties as strain CBS141584.

2. *Lactobacillus rhamnosus* strain according to claim 1, in frozen, dried or in freeze dried form.

3. An antimicrobial and/or antifungal composition comprising the *Lactobacillus rhamnosus* strain as defined in claim 1 or 2.

4. Antimicrobial and/or antifungal composition according to claim 3, further comprising a *Lactobacillus sakei* strain, preferably a *Lactobacillus sakei* strain deposited as CBS141161 .

5. A food product comprising a *Lactobacillus rhamnosus* strain as defined in claim 1 or 2 or an antimicrobial and/or antifungal composition according to claim 3 or 4.

6. A food product according to claim 5, comprising an amount of *Lactobacillus rhamnosus* strain and/or an amount of *Lactobacillus sakei* strain, which is effective for imparting antimicrobial and/or antifungal properties to the food product.

7. A food product according to claim 5 or claim 6, wherein the food product is a dairy product, preferably a mesophilic or thermophilic fermented milk product, such as yogurt or sour cream.

8. Method for manufacturing a food product comprising adding the *Lactobacillus rhamnosus* strain as defined in claim 1 or 2, or an antimicrobial and/or antifungal composition according to claim 3 or 4 during manufacture of the food product.

9. Method according to claim 8, wherein the amount of the *Lactobacillus rhamnosus* strain is sufficient for imparting antimicrobial and/or antifungal properties to the food product.

10. Method according to claim 8 or claim 9, wherein the food product is a dairy product, preferably a mesophilic or thermophilic fermented milk product, such as yogurt or sour cream.

11. Method according to any of the claims 8 to 10, wherein the *Lactobacillus rhamnosus* strain is added before or during a step of fermenting the food product.

12. Method according to claim 11, wherein the method comprises fermenting milk with a starter culture comprising at least one strain of the genera selected from *Lactobacillus, Streptococcus, Lactococcus* and *Leuconostoc.*

13. Use of the *Lactobacillus rhamnosus* strain as defined in claim 1 or 2 for providing an antifungal effect in dairy products.

14. Use of the *Lactobacillus rhamnosus* strain as defined in claim 1 or 2 for providing an antifungal effect in silage.

15. Use according to claim 13, wherein the dairy product is a mesophilic or thermophilic fermented milk product, such as yogurt or sour cream.

## Patentansprüche

1. *Lactobacillus rhamnosus*-Stamm, hinterlegt unter CBS141584, oder davon abgeleitete Mutanten, wobei die Mutanten die gleichen oder verbesserte antimikrobielle, antimykotische oder Anti-Hefe-Eigenschaften wie Stamm CBS141584 besitzen.

2. *Lactobacillus rhamnosus*-Stamm nach Anspruch 1, in gefrorener, getrockneter oder gefriergetrocketer Form.

3. Antimikrobielle und/oder antimykotische Zusammensetzung, umfassend den *Lactobacillus rhamnosus-*Stamm gemäß Anspruch 1 oder 2.

4. Antimikrobielle und/oder antimykotische Zusammensetzung nach Anspruch 3, ferner umfassend einen *Lactobacillus sakei*-Stamm, vorzugsweise einen unter CBS141161 hinterlegten *Lactobacillus sakei*-Stamm.

5. Lebensmittelprodukt, umfassend einen *Lactobacillus rhamnosus*-Stamm gemäß Anspruch 1 oder 2 oder eine anti¬mikrobielle und/oder antimykotische Zusammensetzung nach Anspruch 3 oder 4.

6. Lebensmittelprodukt nach Anspruch 5, umfassend eine Menge an *Lactobacillus rhamnosus*-Stamm und/oder eine Menge an *Lactobacillus sakei*-Stamm, die wirksam ist, um dem Lebensmittelprodukt antimikrobielle und/oder antimykotische Eigenschaften zu verleihen.

7. Lebensmittelprodukt nach Anspruch 5 oder Anspruch 6, wobei es sich bei dem Lebensmittelprodukt um ein Molkereiprodukt, vorzugsweise ein mesophiles oder thermophiles vergorenes Milchprodukt wie Joghurt oder Sauerrahm, handelt.

8. Verfahren zur Erzeugung eines Lebensmittelprodukts, umfassend Zugeben des *Lactobacillus rhamnosus-*Stamms gemäß Anspruch 1 oder 2 oder einer antimikrobiellen und/oder antimykotischen Zusammensetzung nach Anspruch 3 oder 4 während der Erzeugung des Lebensmittelprodukts.

9. Verfahren nach Anspruch 8, wobei die Menge des *Lactobacillus rhamnosus*-Stamms hinreichend ist, um dem Lebensmittelprodukt antimikrobielle und/oder antimykotische Eigenschaften zu verleihen.

10. Verfahren nach Anspruch 8 oder Anspruch 9, wobei es sich bei dem Lebensmittelprodukt um ein Molkereiprodukt, vorzugsweise ein mesophiles oder thermophiles vergorenes Milchprodukt wie Joghurt oder Sauerrahm, handelt.

11. Verfahren nach einem der Ansprüche 8 bis 10, wobei der *Lactobacillus rhamnosus-Stamm* vor oder bei einem Schritt des Vergärens des Lebensmittelprodukts zugegeben wird.

12. Verfahren nach Anspruch 11, wobei das Verfahren Vergären von Milch mit einer Starterkultur, die wenigstens einen Stamm der Gattungen ausgewählt aus *Lactobacillus, Streptococcus, Lactococcus* und *Leuconostoc* umfasst, umfasst.

13. Verwendung des *Lactobacillus rhamnosus*-Stamms gemäß Anspruch 1 oder 2 zur Bereitstellung einer antimykotischen Wirkung in Molkereiprodukten.

14. Verwendung des *Lactobacillus rhamnosus*-Stamms gemäß Anspruch 1 oder 2 zur Bereitstellung einer antimykotischen Wirkung in Silage.

15. Verwendung nach Anspruch 13, wobei es sich bei dem Molkereiprodukt um ein mesophiles oder thermophiles vergorenes Milchprodukt wie Joghurt oder Sauerrahm handelt.

## Revendications

1. Souche de *Lactobacillus rhamnosus* déposée en tant que CBS141584, ou des mutants dérivés de celle-ci, où lesdits mutants possèdent les mêmes propriétés antimicrobiennes, antifongiques et/ou anti-levure, ou des propriétés améliorées, par rapport à la souche CBS141584.

2. Souche de *Lactobacillus rhamnosus* selon la revendication 1, sous forme congelée, séchée ou lyophilisée.

3. Composition antimicrobienne et/ou antifongique, comprenant la souche de *Lactobacillus rhamnosus* telle que définie selon la revendication 1 ou 2.

4. Composition antimicrobienne et/ou antifongique selon la revendication 3, comprenant en outre une souche de *Lactobacillus sakei,* préférablement une souche de *Lactobacillus sakei* déposée en tant que CBS141161.

5. Produit alimentaire comprenant une souche de *Lactobacillus rhamnosus* telle que définie selon la revendication 1 ou 2, ou une composition antimicrobienne et/ou antifongique selon la revendication 3 ou 4.

6. Produit alimentaire selon la revendication 5, comprenant une quantité d'une souche de *Lactobacillus rhamnosus* et/ou une quantité d'une souche de *Lactobacillus sakei,* qui est efficace pour conférer des propriétés antimicrobiennes et/ou antifongiques au produit alimentaire.

7. Produit alimentaire selon la revendication 5 ou selon la revendication 6, **caractérisé en ce que** le produit alimentaire est un produit laitier, préférablement un produit de lait fermenté mésophile ou thermophile, tel qu'un yaourt ou une crème sure.

8. Méthode de préparation d'un produit alimentaire, comprenant l'addition de la souche de *Lactobacillus rhamnosus* telle que définie selon la revendication 1 ou 2, ou d'une composition antimicrobienne et/ou antifongique selon la revendication 3 ou 4, lors de la fabrication du produit alimentaire.

9. Méthode selon la revendication 8, dans laquelle la quantité de la souche de *Lactobacillus rhamnosus* est suffisante pour conférer des propriétés antimicrobiennes et/ou antifongiques au produit alimentaire.

10. Méthode selon la revendication 8 ou la revendication 9, dans laquelle le produit alimentaire est un produit laitier, préférablement un produit de lait fermenté mésophile ou thermophile, tel qu'un yaourt ou une crème sure.

11. Méthode selon l'une quelconque des revendications 8 à 10, dans laquelle la souche de *Lactobacillus rhamnosus* est ajoutée avant ou pendant une étape de fermentation du produit alimentaire.

12. Méthode selon la revendication 11, **caractérisée en ce que** la méthode comprend la fermentation de lait avec une culture starter comprenant au moins une souche issue des genres choisis parmi *Lactobacillus, Streptococcus, Lactococcus* et *Leuconostoc.*

13. Utilisation de la souche de *Lactobacillus rhamnosus* telle que définie selon la revendication 1 ou 2, afin d'apporter un effet antifongique dans des produits laitiers.

14. Utilisation de la souche de *Lactobacillus rhamnosus* telle que définie selon la revendication 1 ou 2, afin d'apporter un effet antifongique dans de l'ensilage.

15. Utilisation selon la revendication 13, dans laquelle le produit laitier est un produit de lait fermenté mésophile ou thermophile, tel qu'un yaourt ou une crème sure.
